# EUROPEAN PATENT SPECIFICATION

(11) **EP 3 771 448 B1**
(45) Date of publication and mention of the grant of the patent: **15.01.2025**
(21) Application number: 20188678.5
(22) Date of filing: 30.07.2020
(51) Int. Cl.: A61B 17/70, A61F 2/46, A61B 17/88

(54) **VERTEBRAL IMPLANT**
WIRBELIMPLANTAT
IMPLANT VERTÉBRAL

(30) Priority: 31.07.2019 TW 108127156
(43) Date of publication of application: 03.02.2021
(73) Proprietor: Apcore Medical Technology Co., Ltd., Taicang City (CN)
(72) Inventor: Shieh, Jiann-Shing, 114 Taipei City (TW); Lu, Shih-Chun, 114 Taipei City (TW); Luo, Guan-Shin, 114 Taipei City (TW); Lin, Ying-Lien, 114 Taipei City (TW); Lin, Shih Hung, 114 Taipei City (TW); Ting, Po-Yao, 114 Taipei City (TW)
(74) Representative: Aseglio-Gianinet, Romina

(56) References cited:
- DE-A1- 102012 003 247
- US-A1- 2009 177 206
- US-A1- 2018 153 602
- US-B1- 6 716 216

## Description

### BACKGROUND OF THE INVENTION

### 1. Field of the Invention

The present invention relates to a vertebral implant, and in particular to a vertebral implant having a three-dimensional porous device.

### 2. The Prior Arts

In the operation of filling or inserting the medical filler into the bone, the current surgical methods commonly include the following:

When using a mechanical expanding device (such as patents US20110196494, US20110184447, US20100076426, US20070067034, US20060009689, US20050143827, US20220052623, US6354995, US6676665) for bone expanding to generate a space in the bone, the expanding device is taken out after expanding, then a covering device is inserted, and then the operation for filling or stuffing of the medical filler is performed. This type of operation has the following disadvantages: Mechanical expanding devices crush the cancellous bone when expanding and the crushed fragments often fall into the mechanical expanding device, which causes the mechanical expanding device to get stuck, and makes the expanded element unable to be retracted (recovered to a contracted state), this results in that the entire mechanical expanding device is stuck. Thereby, it cannot be retracted from the expanded position.

When a filling expanding device (such as patents US5972015, US6066154, US6235043, US6423083, US6607544, US6623505, US6663647, US6716216) for bone expanding to generate a space in the bone are used, the expanding device is taken out after expanding, then a covering device is inserted, and then the operation for filling or stuffing of the medical filler is performed. Because the filling expanding device mostly puts a balloon into the bone, and uses high pressure to inject liquid (such as water) into the balloon (a variety of balloons are used for different needs), the balloon is inflated to push the cancellous bone into the bone to achieve the purpose of expanding. However, the device or method has many disadvantages, for example, the balloon must be connected to a nozzle, so when the filling of the liquid is under high pressure, it may cause the balloon to fall off from the nozzle, and the balloon may even burst.

Without expanding in advance, the covering device is directly placed into the bone and the medical filler is injected, and the pressure under which the medical filler is injected into the covering device is used to achieve the effect of spreading the bone. Under such a situation, the covering device is both an expanding device and a vertebrae fixation device when infusing the medical filler (such as patents TWI321467, TW201112995, US6248110). Alternatively, the covering device is even abandoned, and a perfusion device is directly used to inject the medical filler into the surgical position to enhance the fixation of the surgical position (such as patent US5514137). This kind of surgery has the following disadvantages: Because no expanding is performed first, the range of perfusion cannot be accurately controlled, so that the direction after finishing perfusion may be different from that originally expected by the doctor, and it is even found that the covering device does not completely support the bone or the medical filler flows around in the bone after the medical filler is injected, and the medical filler may even flow out of the bone, or the concentration of the slurry medical filler may be too thin or the particle is too small, which makes it difficult to support the bone when infusing the medical filler, and greatly reduces the original effect.

The mechanical expanding device can be used as a vertebrae fixation device (such as patents US20120071977, US20110046739, US20100069913, US20100217335, US20090234398, US20090005821). After the mechanical expanding device is implanted into the bone and the bone is opened, the medical filler is injected, the medical filler is allowed to cover the mechanical expanding device, and the mechanical expanding device and the medical filler are left in the human body together after the filling is finished. This kind of surgical method uses no covering device, the flow direction of the medical filler cannot be effectively controlled, and thus it is possible for the medical filler to flow around the bone, and even to flow out of the bone. In addition, the medical filler cannot effectively and completely cover the mechanical expanding device, and the mechanical expanding device may thus slowly contract from a fully expanded state to an incompletely expanded state. As a result, the bones are not completely expanded, which means that the original purpose of the vertebrae fixation device is lost.

US2018153602 discloses systems and methods of delivering and deploying a stent into a curvilinear cavity within a vertebral body or other bony or body structure. In some instances, the system can include an elongate shaft for delivering a self-expanding, cement-directing stent and devices that may be used to perform the steps to deliver and deploy the stent.

DE102012003247 discloses a vertebral stent. The vertebral stent is transported in an elongated mold in an application pipe and fulfills a supporting function in the vertebral body in the elongated mold. The vertebral stent is made of mold memory alloy and partially has a grid- or mesh structure. The vertebral stent has a holding element at its distal end, where the holding element is accessible through the interior of vertebral stent and is positioned in an application device. Another holding element is provided at a proximal end of vertebral stent, in which another application device is positioned.

A covering device can be used to cover the expansion device as a vertebral fixation device (such as patent US 10080595). The covering device can effectively prevent fragments of the crushed cancellous bone from falling into the expansion device during expanding, so that the expansion device can be repeatedly expanded and contracted in the bone for expanding to adjust the direction of expanding or the size of the expanding range, and to control the range of perfusion of the medical filler by the covering device when infusing the medical filler. After the perfusion is finished, the medical filler can completely cover the expansion device. However, the pores of the covering device of this invention are not three-dimensional connecting pores, so the effect of the perfused medical filler and bones to achieve interdigitate is poor. Secondly, the medical filler in the covering device is solid, and its strength is far higher than that of cancellous bone in the bone, which is likely to cause stress concentration, which makes it difficult for bone cells near the implant to grow after surgery.

The present invention uses a three-dimensional porous device to control the perfusion range of the medical filler, and since the three-dimensional porous device has a three-dimensional connecting pore structure, the medical filler subsequently injected is closer to the structure of the cancellous bone and less likely to have stress concentration. In addition, the medical filler flows through the three-dimensional connecting pore structure and contacts the vertebrae, which is more likely to achieve an interdigitate effect. Furthermore, the three-dimensional porous device can apply biodegradable materials, and the medical filler will form countless three-dimensional connecting channels therein with the three-dimensional porous device degraded later in the body, and the bone cells can grow into the medical filler through these connecting channels and form a denser connection with the medical filler.

### SUMMARY OF THE INVENTION

The three-dimensional porous device of the present invention can automatically expand after being compressed, it expands after being placed in a vertebrae in a compressed state, and the three-dimensional porous device is filled with a medical filler. The medical filler interdigitates into the vertebrae by the three-dimensional porous device and is connected to the vertebrae. The three-dimensional porous device can effectively control the flow direction and perfusion range of the medical filler, and prevent the medical filler from running around in the vertebrae. In addition, because the three-dimensional porous device has a three-dimensional connecting pore structure, the injected medical filler will be closer to the structure of the cancellous bone in the vertebrae, and its mechanical performance will be closer to that of cancellous bone. Thus, it is less likely to have problematic stress concentration and is advantageous for bone cell growth after the operation. After the three-dimensional porous device is slowly degraded in the body, multiple connecting channels will be formed in the medical filler to provide bone cell growth, so that the bones and the medical filler are connected closer.

A purpose of the present invention is to provide a vertebral implant having a three-dimensional porous device.

Yet another purpose of the present invention is to provide a vertebral implant having a three-dimensional porous device capable of being compressed and expanded.

A further purpose of the present invention is to provide a vertebral implant having a function of expanding the bone.

Another further purpose of the present invention is to provide a vertebral implant using a three-dimensional porous device to control a flow direction and a perfusion range of a medical filler.

Another purpose of the present invention is to provide a vertebral implant using a three-dimensional porous device to enhance the interdigitate effect of a medical filler.

Yet another purpose of the present invention is to provide a vertebral implant using a compression piece to compress a three-dimensional porous device in advance.

A vertebral implant of the present invention comprises: a stuffing including: a perfusion device having a proximal end being an injection port, the perfusion device having at least one perfusion port; and a three-dimensional porous device covering at least part of the perfusion device and covering the at least one perfusion port, wherein the three-dimensional porous device is capable of automatically expanding after being compressed; a hollow tube having a distal end being connected with the proximal end of the stuffing by a detachable structure; and a medical filler being filled into the three-dimensional porous device via the hollow tube and the perfusion device, wherein the three-dimensional porous device is a three-dimensional connecting pore structure.

The perfusion port may be a perfusion port with any conventional form, such as a groove, a hole, etc. so that the medical filler can be filled into the three-dimensional porous device through the perfusion port.

The three-dimensional porous device can effectively control the flow direction and perfusion range of the medical filler, and prevent the medical filler from running around in the vertebrae, causing uneven perfusion, or even the medical filler flowing out of the vertebrae, causing a risk to the patient, and effectively achieve the interdigitate effect, so that the medical filler and the vertebrae are connected more closely. In addition, the size, porosity, etc. of the three-dimensional connecting pore structure can be adjusted to not only control the overall mechanical strength of the medical filler and the three-dimensional porous device to make its mechanical performance closer to the strength of cancellous bone in the vertebrae for preventing stress concentration and being beneficial to the growth of bone cells after surgery, but also be used to adjust the smoothness of perfusion of the medical filler into the three-dimensional porous device.

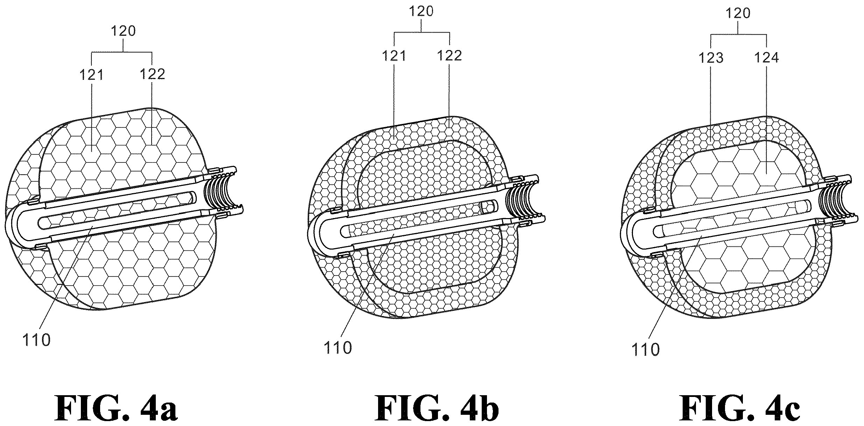

The aforementioned three-dimensional connecting pore structure may further be a three-dimensional connecting pore structure with a fixed pore size (see FIG. 4a), a three-dimensional connecting pore structure with different pore sizes (see FIG. 4c), or a three-dimensional connecting pore structure being hollow inside (see Figure 4b) can be selected with three-dimensional porous devices with appropriate pore sizes according to the different thicknesses of the medical filler or bone looseness. On one hand, the smoothness of the perfusion of the medical filler can be increased. On the other hand, the mechanical performance of the completely formed medical filler and three-dimensional porous device can be adjusted through the selection of the pore size.

The pore size of the three-dimensional porous device may be 10µm to 3000µm.

The three-dimensional porous device may further be an expandable three-dimensional porous device, and the expandable three-dimensional porous device is in a pre-compressed state before entering the vertebrae, and is expandable after being inserted into the vertebrae, so as to facilitate the subsequent perfusion of the medical filler. The three-dimensional porous device can also expand with the filling of the medical filler after the three-dimensional porous device enters the vertebrae, the expandable three-dimensional porous device can be foam, sponge, or any compressible/expandible three-dimensional porous elastomer.

In the three-dimensional porous device described above, the material forming the structure of the three-dimensional porous device may be any conventional biocompatible material, such as polyethylene, polyurethane, polyvinyl alcohol, nylon, silicone, etc., or may further be biodegradable materials, such as polylactic acid, gelatin, alginate, polyglycolic acid, polyhydroxy fatty acid esters, polycaprolactone, etc. In this way, after the three-dimensional porous device and the medical filler are implanted into the human body, the three-dimensional porous device can be slowly degraded in the human body. The structure of the three-dimensional porous device will form multiple connecting channels in the medical filler, and the bone cells can grow into the medical filler through these connecting channels, and be formed to connect and be intertwined with the medical filler more closely. The material of the three-dimensional porous device is preferably a biodegradable material.

In the structure forming the three-dimensional porous device, the curved surface boundary of any pore is composed of the material mentioned above in the shape of filament/thin line, and the diameter of the filament/thin line (hereinafter referred to as the structure diameter) may be 10µm~3000µm. Through the selection of the structure diameter, the diameter of the connecting channel left by the three-dimensional porous device degraded in the body can be controlled. The diameter of the structure of the three-dimensional porous device therein preferably is 10µm~ 1000µm, while 100µm~500µm is preferred for bone cells to grow easily.

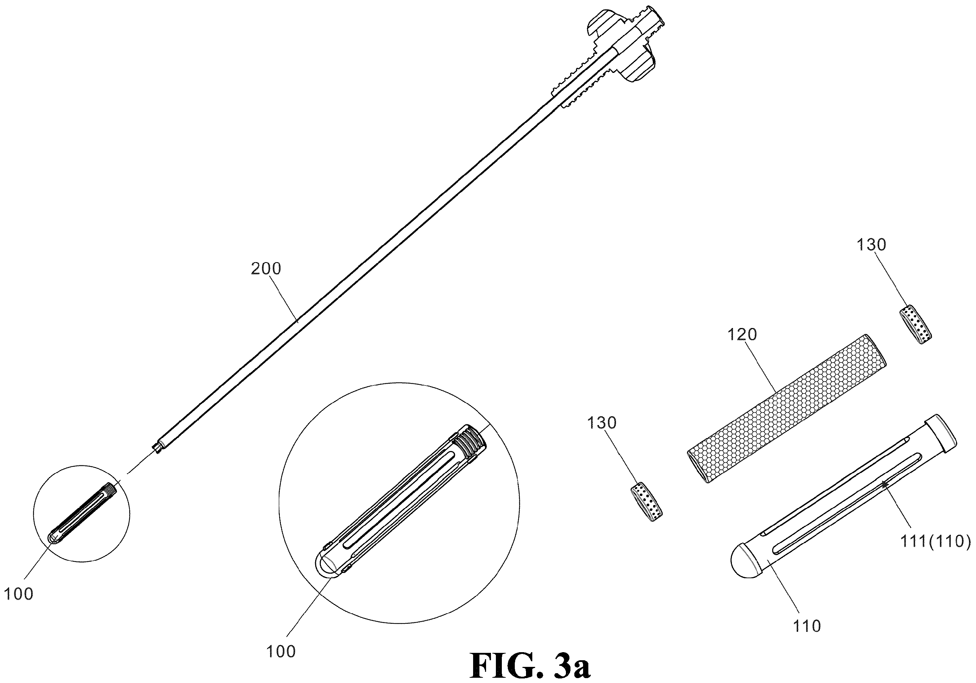

The stuffing described above may further include at least one fixing piece (shown as reference numeral 130 in FIG. 3a), by using the at least one fixing piece, for the operator, a farther end and a closer end of the three-dimensional porous device is fixed to the distal end and the proximal end respectively of the perfusion device. In this way, the three-dimensional porous device can be prevented from being displaced due to the excessive pressure of the medical filler and causing the medical filler to overflow into the unexpected filling direction that causes a risk or death of a patient.

The above-mentioned detachable structure of the proximal end of the hollow tube and the stuffing may be any conventional detachable structure. After the medical filler is filled into the three-dimensional porous device through the hollow tube and the perfusion device, the detachable structure can be used to detach the stuffing from the hollow tube, and to leave the stuffing in the vertebrae. The detachable mechanism can be any conventional detachable structure, such as a screw, engaging, fastening, or locking detachable structures, wherein the detachable structure of the screw is preferred.

The above-mentioned medical filler may be any conventional consolidable and slurry medical filler, such as bone cement. The above-mentioned consolidable and slurry medical filler preferably is a medical filler with osteo-conductive and/or osteo-inductive materials added thereto, such as the conventional hydroxyapatite, calcium phosphate-based bone fillers. Besides, it is also preferred to add bone-leading medical fillers, such as the conventional SrHA-based medical filler.

The vertebral implant may further include a hollow guide tube, a proximal end of the hollow guide tube has an introduction port, the stuffing enters the hollow guide tube from the introduction port, and is placed in the vertebrae under the guidance of the hollow guide tube.

The three-dimensional porous device may further include a compression piece, the compression piece is arranged around the three-dimensional porous device, and the compression piece substantially covers the three-dimensional porous device before the three-dimensional porous device is placed in the vertebrae to compress the three-dimensional porous device. After the three-dimensional porous device enters the hollow guide tube through the introduction port, the compression piece is detached from the three-dimensional porous device to make the three-dimensional porous device expand.

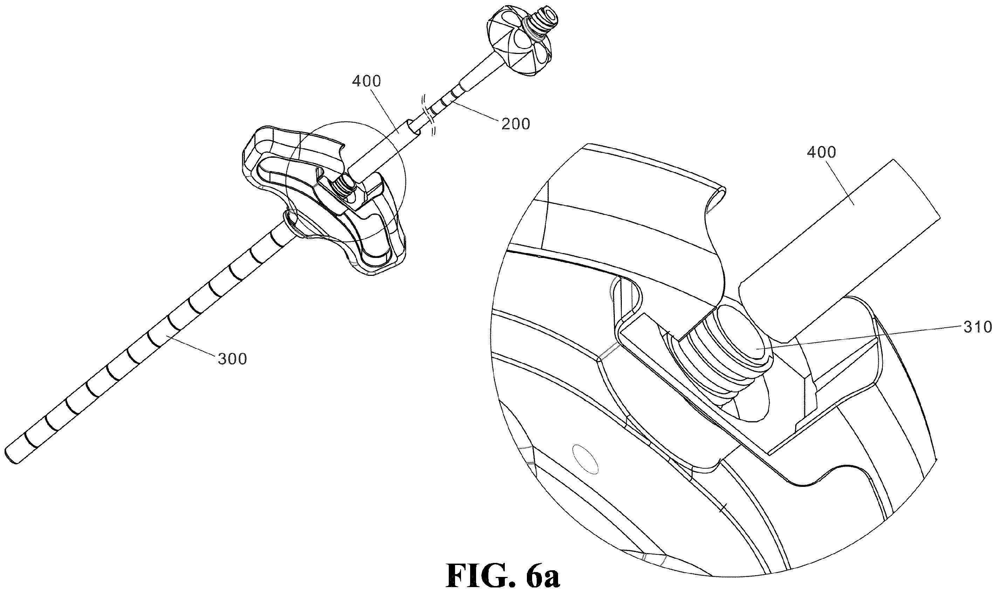

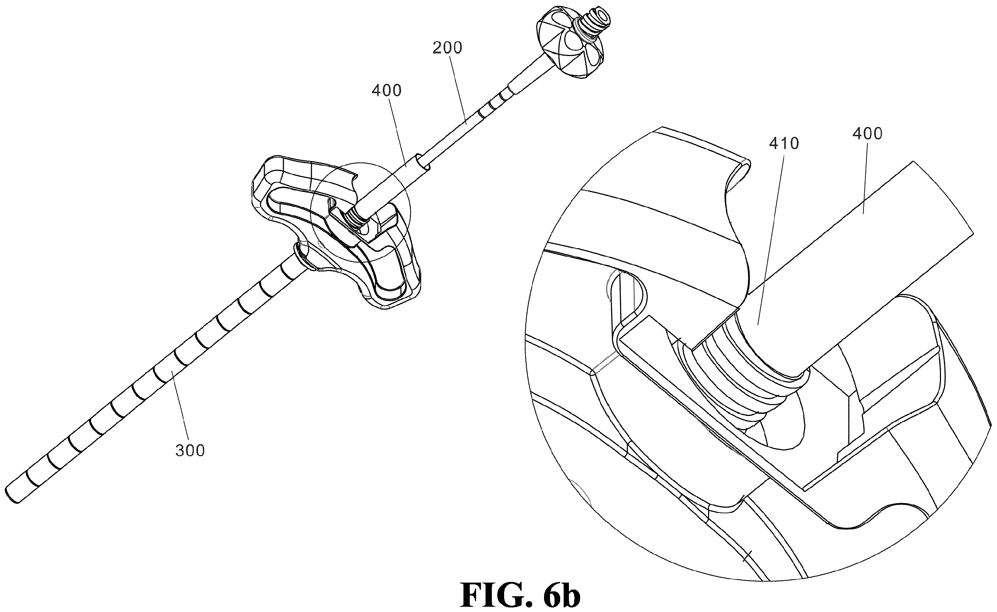

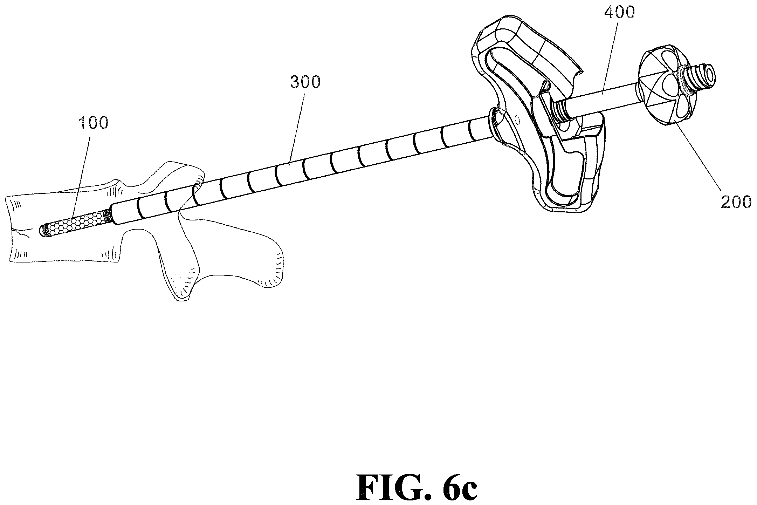

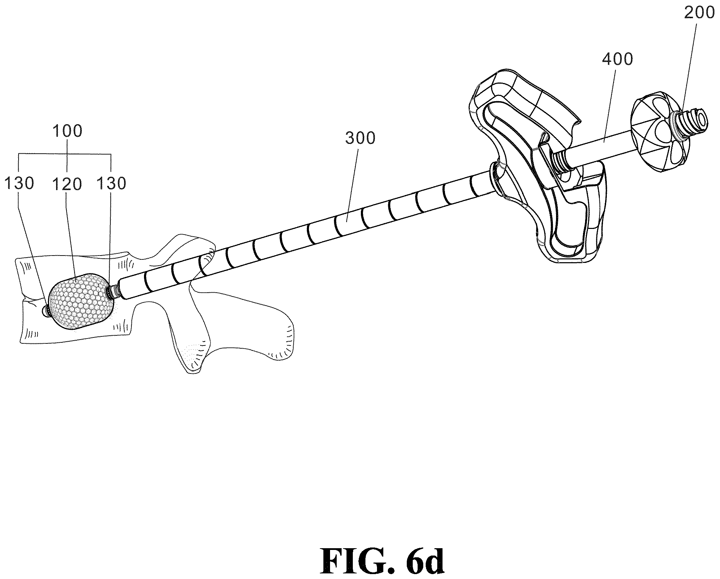

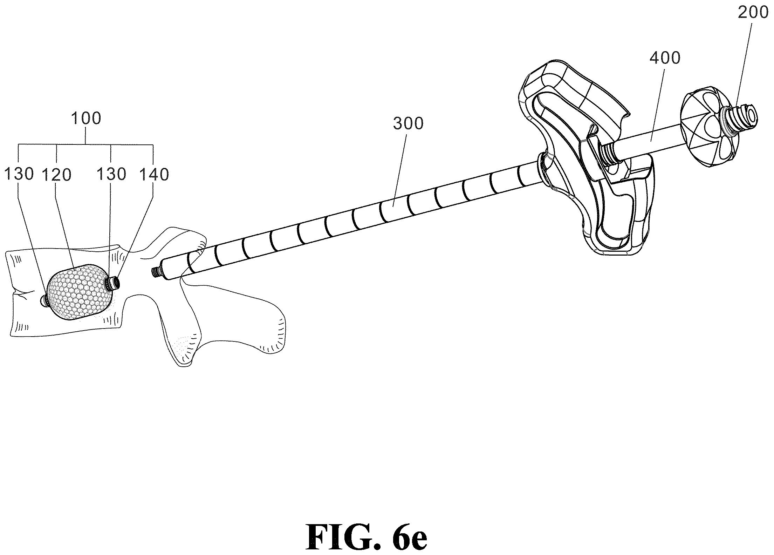

The compression piece may be a sleeve, and the outer diameter of the sleeve is substantially larger than the inner diameter of the introduction port (as shown in FIGs. 6a, 6b). Therefore, when the stuffing and the hollow tube enter the hollow guide tube, the compression piece will automatically detach from the stuffing and stay outside the proximal end of the hollow guide tube (as shown in FIGs. 6a, 6b, 6c). After the stuffing protrudes from the distal end of the hollow guide tube, the three-dimensional porous device expands (as shown in FIG. 6d). After the three-dimensional porous device is filled with the medical filler, the stuffing is removed from the hollow tube by using the detachable structure, and the stuffing is left in the vertebrae (as shown in FIG. 6e).

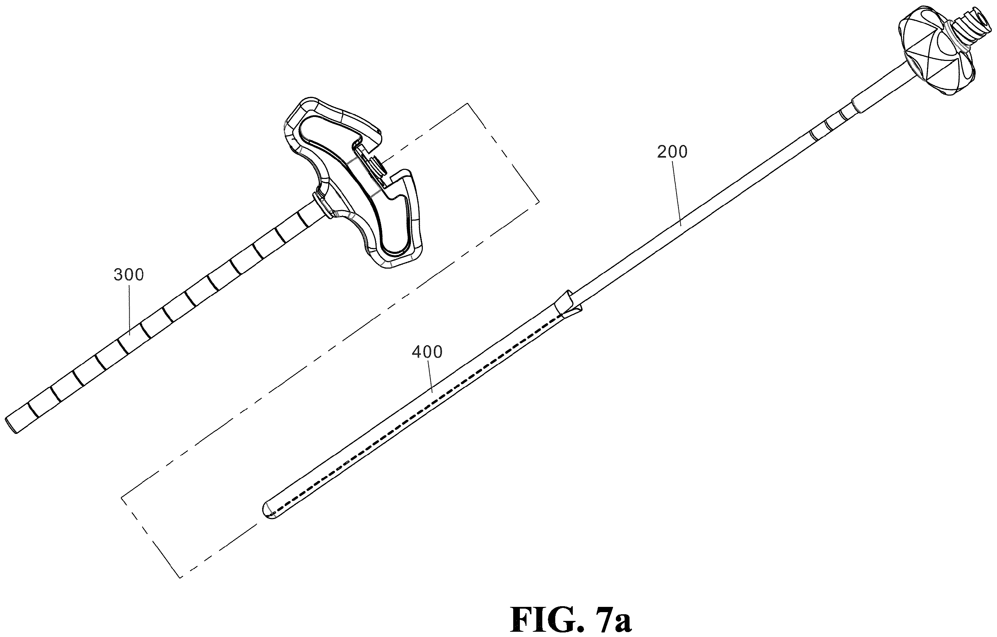

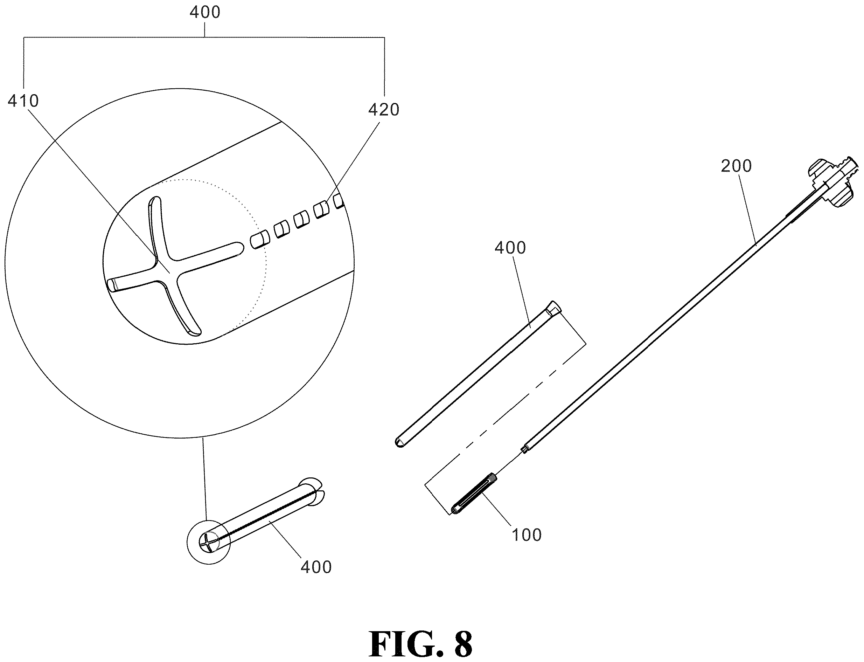

The compression piece can be a piece with easy-to-rupture lines on one side and a pre-cut groove/pre-cut hole/pre-cut line at the distal end (as shown in Fig. 7a, Fig. 8). After the stuffing and the hollow tube enters the hollow guide tube, the easy-to-rupture line and the pre-cut groove/pre-cut hole/pre-cut line are used to rupture the compression piece and retract the hollow guide tube. After the stuffing extends out of the distal end of the hollow guide tube, the three-dimensional porous device will expand. After the medical filler is filled into the three-dimensional porous device, the stuffing is removed from the hollow tube by using the detachable structure, and the stuffing is left in the vertebrae.

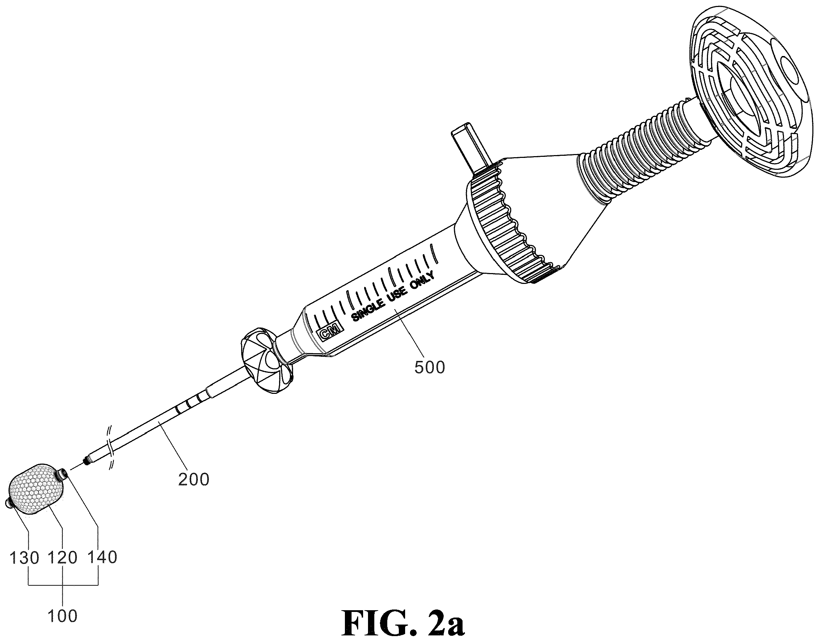

The vertebral implant mentioned above further includes an injector, a distal end of the injector is connected to the proximal end of the hollow tube (as shown in FIG. 2a), and the medical filler is filled into the three-dimensional porous device by the injector.

The vertebral implant may further include an extension tube, a distal end of the extension tube, and a proximal end of the hollow tube are connected in a detachable manner, a proximal end of the extension tube and a distal end of the injector are connected in a detachable manner. The medical filler can fill the medical filler into the three-dimensional porous device by the injector. The detachable method can be any conventional detachable method, such as engaging, locking, screw, etc.

The vertebral implant may further include a blocking device, the blocking device is used to connect the proximal end of the stuffing after the medical filler is filled into the three-dimensional porous device, and the medical filler flowing out from the injection port is blocked by the blocking device. The connection between the blocking device and the proximal end of the stuffing can be any conventional connection method, such as: screw, engaging, locking, buckle, etc. The connection method therein preferably is screw or engaging.

### BRIEF DESCRIPTION OF THE DRAWINGS

The present invention will be apparent to those skilled in the art by reading the following detailed description of a preferred embodiment thereof, with reference to the attached drawings, in which:
FIG. 1a illustrates a diagram of a vertebral implant according to a preferred specific embodiment of the present invention.
FIG. 1b illustrates a diagram of a vertebral implant with a stuffing detached from a hollow tube according to the present invention.
FIG. 2a illustrates a diagram of a perfusion of a vertebral implant according to a preferred specific embodiment of the present invention.
FIG. 2b illustrates a diagram of the perfusion of a vertebral implant according to another specific embodiment of the present invention.
FIG. 3a illustrates an exploded diagram of a stuffing of a vertebral implant according to the present invention.
FIG. 3b illustrates an enlarged cross-sectional view of a stuffing of a vertebral implant according to the present invention.
FIGs. 4a-4c illustrate three specific examples of a three-dimensional porous device of a vertebral implant according to preferred embodiments of the present invention.
FIG. 5a illustrates a diagram of a specific example of a compression piece of a vertebral implant according to the present invention.
FIG. 5b illustrates an exploded diagram of a specific example of a combination of a compression piece and a hollow guide tube of a vertebral implant according to the present invention.
FIGs. 6a-6e illustrate a diagram of surgical steps with combination of a compression piece and a hollow guide tube of a vertebral implant according to the present invention.
FIG. 7a illustrates a compression piece of a vertebral implant according to another preferred embodiment of the present invention.
FIG. 7b illustrates a cross-sectional view of a compression piece of a vertebral implant according to the present invention.
FIG. 8 illustrates an enlarged diagram of a compression piece of a vertebral implant according to the present invention.
FIGs. 9a-9b illustrate diagrams of surgical steps with a vertebral implant according to the present invention.
FIG. 10 illustrates a diagram of another preferred surgical perfusion of a vertebral implant according to the present invention.
FIG. 11 illustrates a diagram of the completion of surgical operation of a vertebral implant according to the present invention.

### DETAILED DESCRIPTION OF THE PREFERRED EMBODIMENT

The following description with drawings and element symbols provides more detail of the embodiment of the present invention, so that those skilled in the art can implement the present invention after studying this specification.

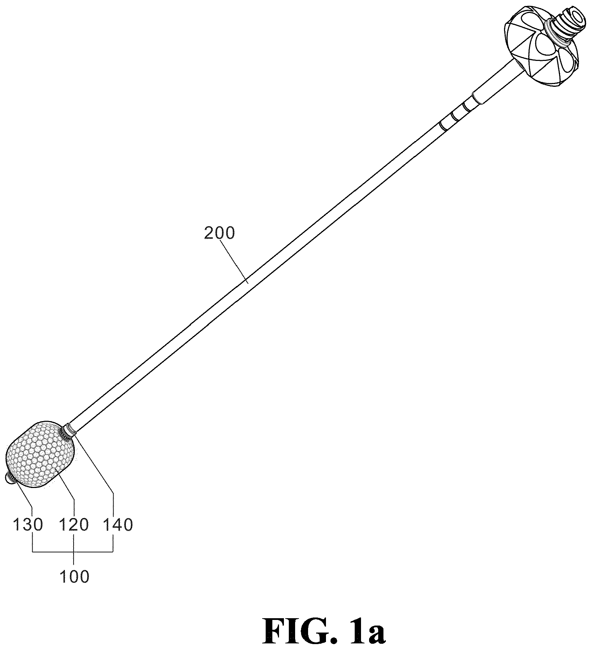

FIG. 1a illustrates a diagram of a vertebral implant according to a preferred specific embodiment of the present invention. An end closer to the operator is a proximal end, and an end farther from the operator is a distal end. A stuffing 100 uses a fixing piece 130 to fix the proximal and distal ends of a three-dimensional porous device 120 to the proximal and distal ends of a perfusion device 110 (not shown in FIG. 1a, see FIG. 3a) to prevent the three-dimensional porous device 120 from being displaced because the perfusion pressure of the medical filler (not shown in FIG. 1a) is too large, which causes the medical filler to overflow in an unexpected filling direction and cause the patient possible paralysis or death. The proximal end of the stuffing 100 is connected to a hollow tube 200 by a detachable structure 140, so as to remove the stuffing 100 from the hollow tube 200 and leave the stuffing 100 in the vertebrae after perfusion of the medical filler.

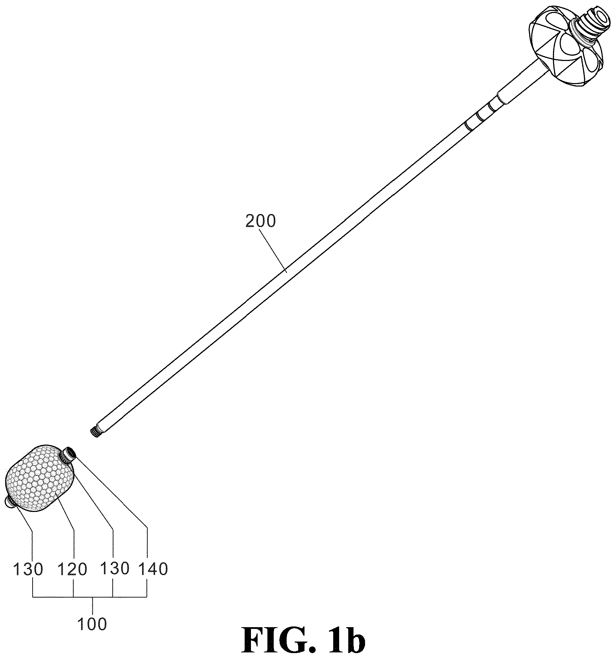

FIG. 1b illustrates a diagram of a vertebral implant with the stuffing 100 detached from the hollow tube 200 according to the present invention. After the medical filler is filled into the stuffing 100, the detachable structure 140 is used to remove the stuffing 100 from the hollow tube 200, and leave the stuffing 100 in the vertebrae.

FIG. 2a illustrates a diagram of the perfusion of a vertebral implant according to a preferred specific embodiment of the present invention. The stuffing 100 is used to fix the three-dimensional porous device 120 to a perfusion device 110 (not shown in FIG. 2a, refer to FIG. 3a) through a fixing piece 130 to avoid the displacement of the three-dimensional porous device 120 due to the excessive pressure of the medical filler (not shown in FIG. 2a), which causes the medical filler to overflow in an unexpected filling direction. The proximal end of the hollow tube 200 connects an injector 500 remotely, the injector 500 is used to fill the medical filler (not shown in FIG. 2a) into the stuffing 100, and the proximal end of the stuffing 100 is connected to the hollow tube 200 by the detachable structure 140 to facilitate removing the stuffing 100 from the hollow tube 200 and leaving the stuffing 100 in the vertebrae after the perfusion of the medical filler.

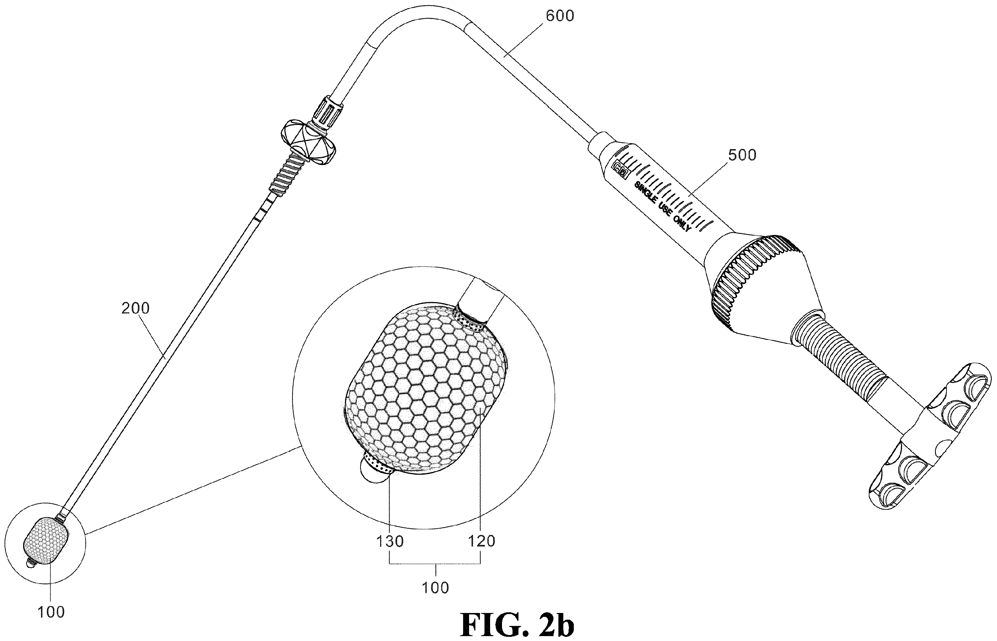

FIG. 2b illustrates a diagram of the perfusion of a vertebral implant according to another specific embodiment of the present invention. A distal end of an extension tube 600 is connected to the hollow tube 200, and a proximal end of extension tube 600 is remotely connected to the injector 500. The extension tube 600 makes it easier for the operator to operate the injector 500 to fill the medical filler into the stuffing 100.

FIG. 3a illustrates an exploded diagram of the stuffing 100 of a vertebral implant according to the present invention. The stuffing 100 is used to fix the proximal end and the distal end of the three-dimensional porous device 120 to the proximal end and the distal end of the perfusion device 110 respectively by the fixing piece 130, so as to prevent the three-dimensional porous device 120 from being displaced due to the excessive pressure of the perfusion of the medical filler, which causes the medical filler to overflow in an unexpected filling direction. The medical filler can be filled into the three-dimensional porous device 120 by a perfusion port 111 of the perfusion device 110.

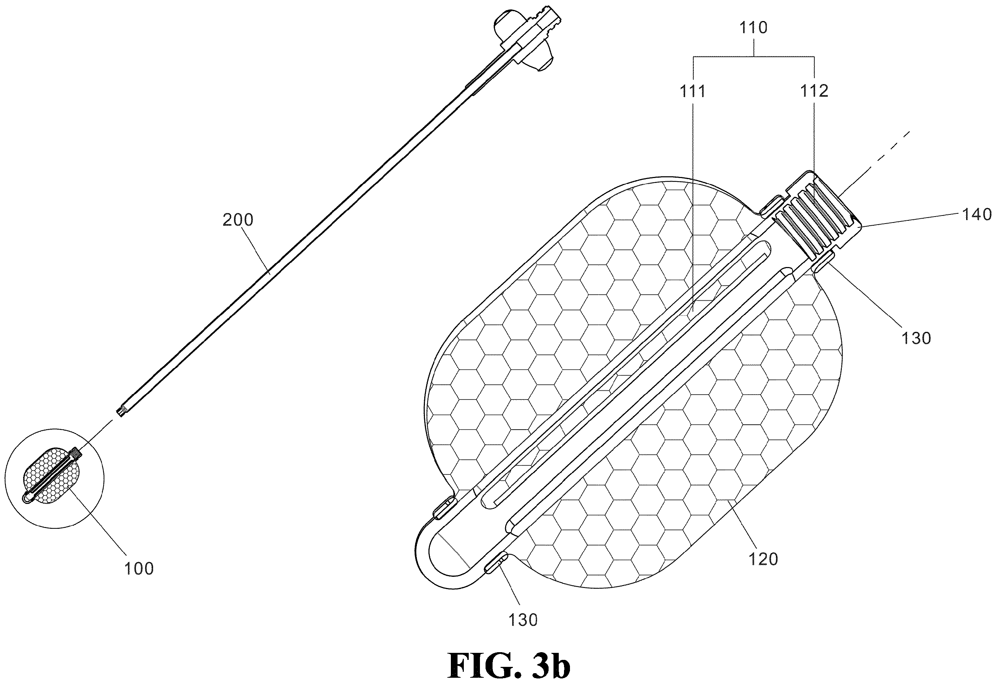

FIG. 3b illustrates an enlarged cross-sectional view of the stuffing 100 of a vertebral implant according to the present invention. The stuffing 100 is used to fix the three-dimensional porous device 120 to the perfusion device 110 by the fixing piece 130, so as to prevent the three-dimensional porous device 120 from being displaced due to the excessive pressure of the perfusion of the medical filler, which causes the medical filler to overflow in an unexpected filling direction. The medical filler is injected from the injection port 112 of the perfusion device 110 and filled into the three-dimensional porous device 120 through the injection port 112 of the perfusion device 110.

FIGs. 4a-4c illustrate three specific examples of the three-dimensional porous device 120 of a vertebral implant according to preferred embodiments of the present invention. FIG. 4a shows the three-dimensional porous device 120 having a three-dimensional connecting pore structure with a uniform size of pore 121. The pressure of the medical filler, and the smoothness of flow can be adjusted by controlling the size of the pore 121 of the three-dimensional porous device 120. The material of the structure 122 of the three-dimensional porous device 120 is biodegradable. After the three-dimensional porous device 120 and the medical filler (not shown) are implanted in the human body, the structure 122 of the three-dimensional porous device 120 will slowly degrade in the human body and form multiple connecting channels in the medical filler, bone cells can grow into the medical filler through these connecting channels, and a tighter intertwined connection with the medical filler is formed.

FIG. 4b shows a three-dimensional porous device 120 with a hollow interior and a three-dimensional connecting pore structure with uniformly sized pores 121 on the periphery. In this way, the medical filler being filled into the three-dimensional porous device 120 may have better fluidity and smoothness, and the flow direction of the medical filler can be controlled through the three-dimensional porous device 120 with the three-dimensional connecting pore structure, and the interdigitate effect of the medical filler is increased, so that the connection between the three-dimensional porous device 120 and the vertebrae is closer.

FIG. 4c shows the three-dimensional porous device 120 having a three-dimensional connecting pore structure with different porosities. An inner layer 124 has a larger porosity and an outer layer 123 has a smaller porosity, the three-dimensional porous device 120 effectively achieves bone interdigitate effect, and the flow direction of the medical filler can be controlled/limited, so as to avoid excessive overflow of the medical filler, which may cause danger to the patient.

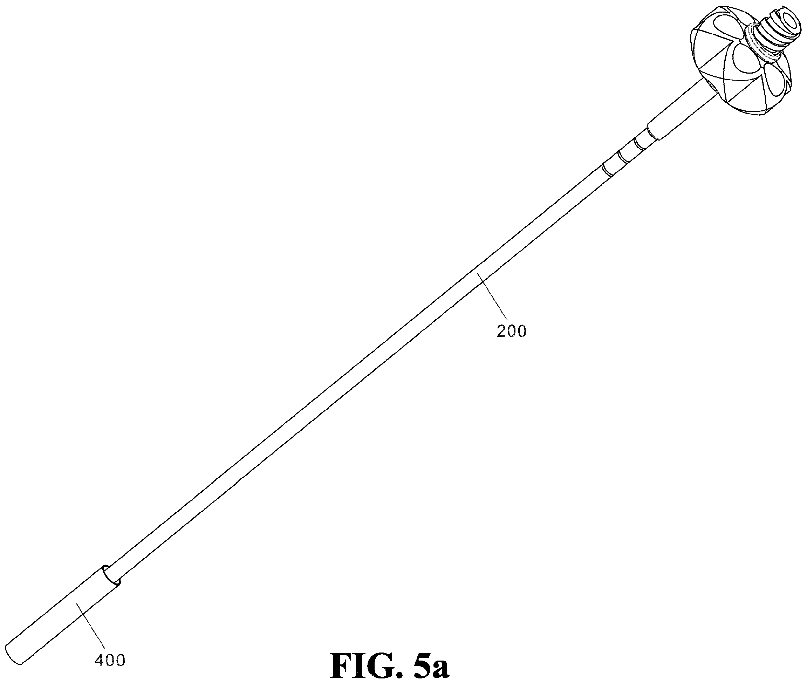

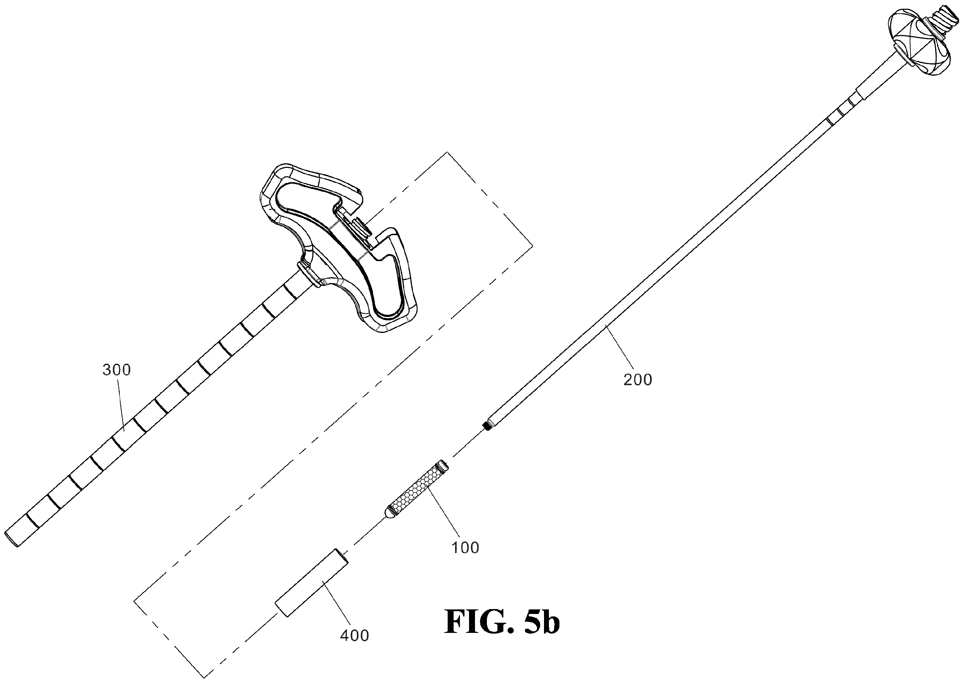

FIG. 5a illustrates a diagram of a specific example of a compression piece 400 of a vertebral implant according to the present invention. The compression piece 400 is arranged around the three-dimensional porous device 120 (not shown, refer to FIG. 5b), and the three-dimensional porous device 120 is covered before being placed in the vertebrae, so as to compress the three-dimensional porous device 120.

FIG. 5b illustrates an exploded diagram of a specific example of a combination of the compression piece 400 and a hollow guide tube 300 of a vertebral implant according to the present invention. The compression piece 400 is arranged around the three-dimensional porous device 120, and the three-dimensional porous device 120 is covered before being injected into the vertebrae to compress the three-dimensional porous device 120. The proximal end of the hollow guide tube 300 has an introduction port 310 (not shown, refer to FIG. 6a) , the stuffing 100 enters the hollow guide tube 300 by way of the introduction port 310 and enters the vertebrae through the guide of hollow guide tube 300.

FIGs. 6a-6e illustrate a diagram of surgical steps with a combination of a compression piece 400 and a hollow guide tube 300 of a vertebral implant according to the present invention. As shown in FIG. 6a, the hollow tube 200, the compression piece 400, and the hollow guide tube 300 are shown. The stuffing 100 is aligned with the introduction port 310 on the proximal end of the hollow guide tube 300. As shown in FIG. 6b, the hollow tube 200 and stuffing 100 (not shown) enter the hollow guide tube 300 from the proximal end of the hollow guide tube 300. The compression piece 400 is left outside the introduction port 310 at the proximal end of the hollow guide tube 300 because the outer diameter of the compression piece 400 is greater than the inner diameter of the introduction port 310. As shown in FIG. 6c, the stuffing 100 extends from the distal end of the hollow guide tube 300, and extends out of the hollow guide tube 300 into the vertebrae, and the compression piece 400 is left outside the introduction port 310 at the proximal end of the hollow guide tube 300. The length of the compression piece 400 can be used to control the stuffing 100 and the hollow tube 200 to extend as far as the distal end of the hollow guide tube 300 as a moderate length (that is, the depth of the stuffing 100 entering the vertebrae is a moderate depth). As shown in FIG. 6d: After the stuffing 100 extends out of the distal end of the hollow guide tube 300 and enters the vertebrae, the three-dimensional porous device 120 of the stuffing 100 is automatically expanded without being restricted by the compression piece 400. As shown in Fig. 6e, after the medical filler is filled to the stuffing 100 via the hollow tube 200, the detachable structure 140 is used to detach the stuffing 100 from the hollow tube 200 and leave the stuffing 100 in the vertebrae.

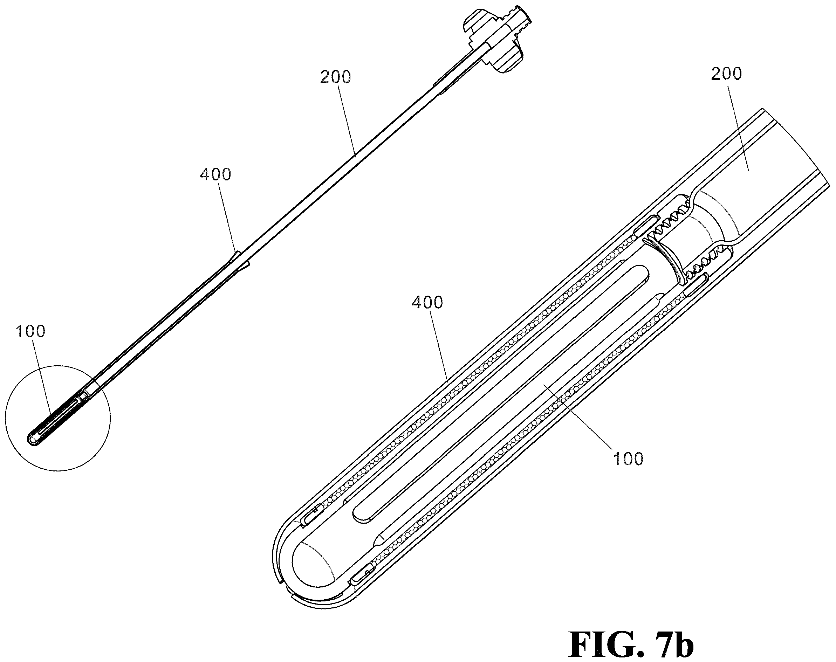

FIG. 7a illustrates the compression piece 400 of a vertebral implant according to another preferred embodiment of the present invention. The compression piece 400 covers the stuffing 100 (not shown, refer to FIG. 7b), that is, part of the hollow tube 200 is used to compress the three-dimensional porous device 120 (not shown, refer to FIG. 7b), so that the stuffing 100 can enter the hollow guide tube 300. The side of the compression piece 400 has an easy-to-rupture line 420 (see Fig.8). After at least part of the compression piece 400 and the stuffing 100 therein enter the hollow guide tube 300 from the introduction port 310, the compression piece 400 can be ruptured apart by the easy-to-rupture line 420 and removed from the stuffing 100, that is, the periphery of hollow tube 200, so that the three-dimensional porous device 120 will not be limited by the compression piece 400 after extending out of the distal end of the hollow guide tube 300.

FIG. 7b illustrates a cross-sectional view of the compression piece 400 of a vertebral implant according to the present invention. The compression piece 400 is arranged outside the stuffing 100 and covers and compresses the three-dimensional stuffing 100, so that the three-dimensional porous device 120 can easily enter the vertebrae through the hollow guide tube 300.

FIG. 8 illustrates an enlarged diagram of a compression piece 400 of a vertebral implant according to the present invention. The compression piece 400 is allowed to be easily ruptured apart and removed from the periphery of the stuffing 100 after entering the hollow guide tube 300 by the side easy-to-rupture line 420 and a remote pre-cut line 410, so that the three-dimensional porous device 120 expands after extending from the distal end of the hollow guide tube 300.

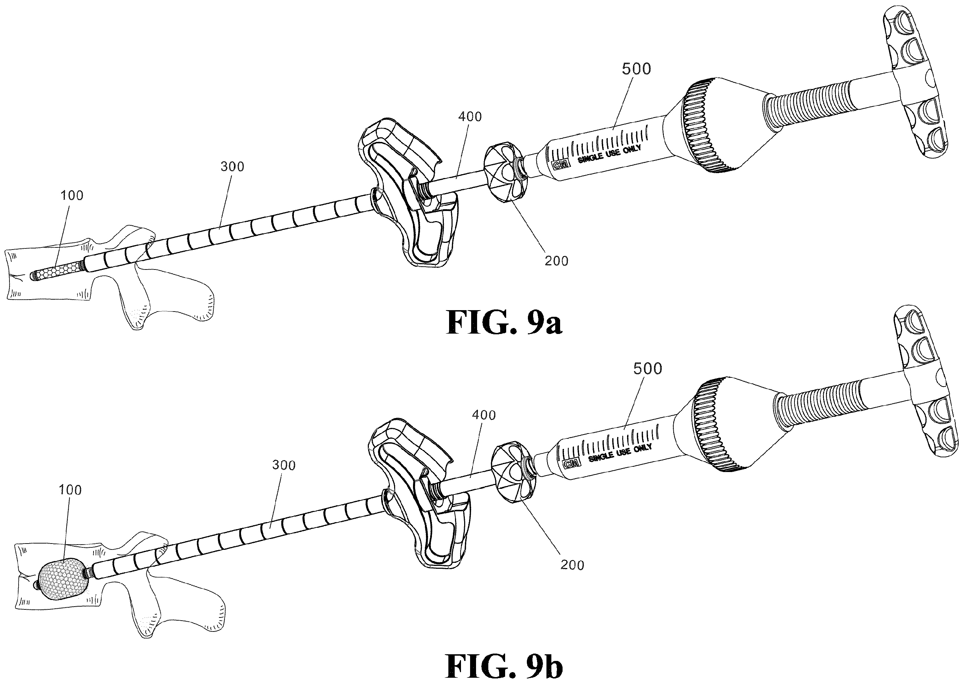

FIGs. 9a-9b illustrate diagrams of surgical steps (not claimed) with a vertebral implant according to the present invention. As shown in FIG. 9a, the stuffing 100 is placed in the vertebrae by the guide of the hollow guide tube 300. As shown in FIG. 9b, after the stuffing 100 enters the vertebrae, the three-dimensional porous device 120 is expanded because the restriction of the compression piece 400 is removed, and then the medical filler is filled to the stuffing 100 through the hollow tube 200 by the injector 500.

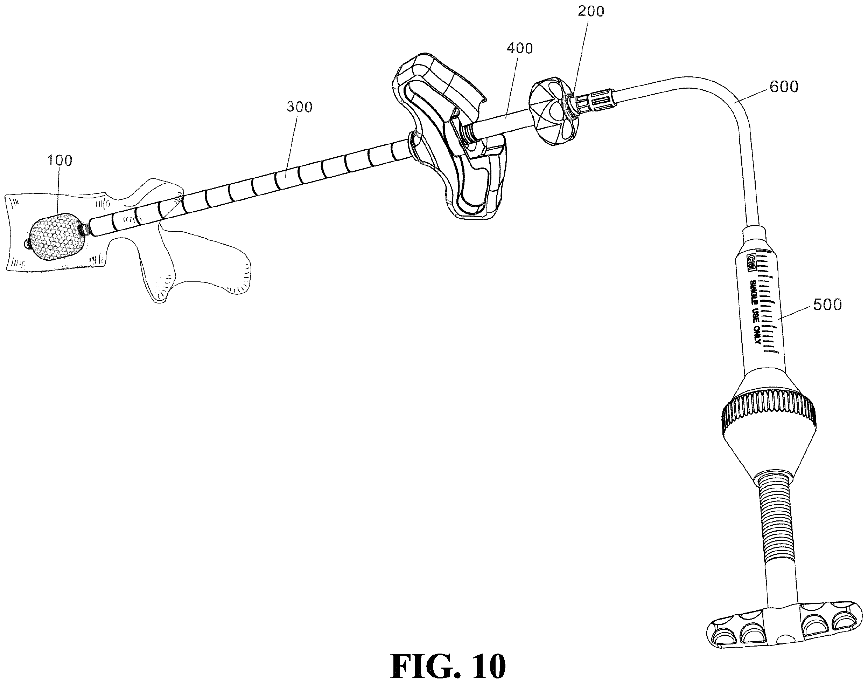

FIG. 10 illustrates a diagram of another preferred surgical perfusion (not claimed) of a vertebral implant according to the present invention. The hollow tube 200 and the injector 500 are connected to an angled extension tube 600, so that the operator can fill the medical filler more conveniently and handily to the stuffing 100.

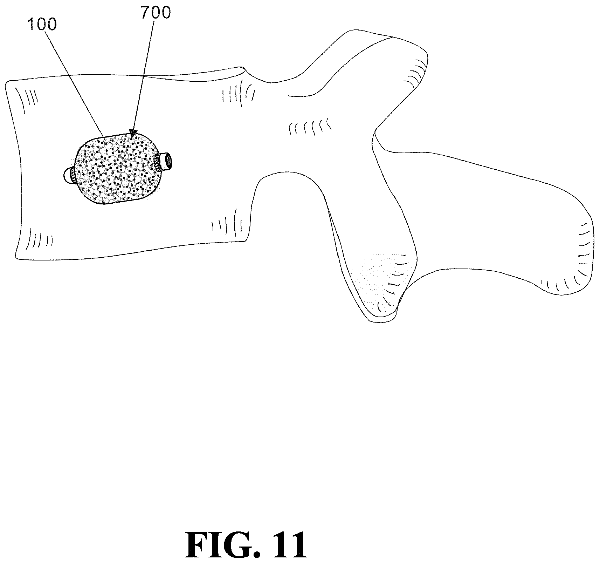

FIG. 11 illustrates a diagram of the completion of surgical operation (not claimed) of a vertebral implant according to the present invention. After filling the stuffing 100 with the medical filler 700, the stuffing 100 is detached from the hollow tube 200 through the detachable structure 140, and the stuffing 100 is left in the vertebrae.

## Claims

1. A vertebral implant comprising:
a stuffing (100) including:
a perfusion device (110) having a proximal end being an injection port (112), the perfusion device (110) having at least one perfusion port (111); and
a three-dimensional porous device (120) covering at least part of the perfusion device (110) and covering the at least one perfusion port (111;
a hollow tube (200) having a distal end being connected with the proximal end of the stuffing (100) by a detachable structure (140); and
a medical filler (700) being filled into the three-dimensional porous device (120) via the hollow tube (200) and the perfusion device (110);
**characterized in that** the three-dimensional porous device (120) is a three-dimensional connecting pore structure, and **in that** the three-dimensional porous device (120) is capable of automatically expanding after being compressed.

2. The vertebral implant of claim 1, **characterized in that** the stuffing (100) further includes at least one fixing piece (130) for fixing the three-dimensional porous device (120) to the perfusion device (110).

3. The vertebral implant of claim 1, **characterized in that** the detachable structure (140) is a detachable screw structure, a detachable engaging structure or a detachable fastening structure.

4. The vertebral implant of claim 1, **characterized in that** a material of the three-dimensional porous device (120) is a biocompatible material.

5. The vertebral implant of claim 4, **characterized in that** the biocompatible material is polyethylene, polyurethane, polyvinyl alcohol, nylon or silicone.

6. The vertebral implant of claim 1, **characterized in that** a material of the three-dimensional porous device (120) is a biodegradable material.

7. The vertebral implant of claim 6, **characterized in that** the biodegradable material is polylactic acid, gelatin, alginate, polyglycolic acid, polyhydroxy fatty acid ester or polyguilide.

8. The vertebral implant of claim 1, **characterized in that** a hole size of the three-dimensional porous device (120) is 10µm-3000µm.

9. The vertebral implant of claim 1, **characterized in that** the medical filler (700) is a slurry medical filler.

10. The vertebral implant of claim 1, **characterized in that** the vertebral implant further includes an injector (500) having a distal end connected to the proximal end of the hollow tube (200), and the medical filler (700) is filled into the three-dimensional porous device (120) by the injector (500).

11. The vertebral implant of claim 1, **characterized in that** the vertebral implant further includes an injector (500) and an extension tube (600), a distal end of the extension tube (600) and the proximal end of the hollow tube (200) are connected detachably, a proximal end of the extension tube (600) is connected to a distal end of the injector (500), the medical filler (700) is injected into the three-dimensional porous device (120) by the injector (500).

12. The vertebral implant of claim 1, **characterized in that** the vertebral implant further includes a hollow guide tube (300), a proximal end of the hollow guide tube (300) has an introduction port (310), the stuffing (100) enters the hollow guide tube (300) from the introduction port (310) and is inserted into vertebrae by guidance of the hollow guide tube (300).

13. The vertebral implant of claim 12, **characterized in that** the vertebral implant further includes a compression piece (400), the compression piece (400) covers and compresses the three-dimensional porous device (120) before the three-dimensional porous device (120) is inserted into the vertebrae, and detaches from the three-dimensional porous device (120) after the three-dimensional porous device (120) enters the hollow tube (200).

## Patentansprüche

1. Wirbelimplantat, umfassend:
eine Füllung (100), umfassend:
eine Perfusionsvorrichtung (110) aufweisend ein proximales Ende, das eine Injektionsöffnung (112) ist, wobei die Perfusionsvorrichtung (110) mindestens eine Perfusionsöffnung (111) aufweist; und
eine dreidimensionale poröse Vorrichtung (120), die zumindest einen Teil der Perfusionsvorrichtung (110) abdeckt und die mindestens eine Perfusionsöffnung (111) abdeckt;
ein Hohlrohr (200) aufweisend ein distales Ende, das mit dem proximalen Ende der Füllung (100) mittels einer lösbaren Struktur (140) verbunden ist; und
ein medizinisches Füllmaterial (700), das über das Hohlrohr (200) und die Perfusionsvorrichtung (110) in die dreidimensionale poröse Vorrichtung (120) eingefüllt wird;
**dadurch gekennzeichnet, dass** die dreidimensionale poröse Vorrichtung (120) eine dreidimensionale, Verbindung-Porenstruktur ist und dass die dreidimensionale poröse Vorrichtung (120) in der Lage ist, sich automatisch zu verbreiten, nachdem sie komprimiert wird.

2. Wirbelimplantat nach Anspruch 1, **dadurch gekennzeichnet, dass** die Füllung (100) ferner mindestens ein Befestigungsteil (130) umfasst, um die dreidimensionale poröse Vorrichtung (120) an der Perfusionsvorrichtung (110) zu befestigen.

3. Wirbelimplantat nach Anspruch 1, **dadurch gekennzeichnet, dass** die lösbare Struktur (140) eine lösbare Schraubenstruktur, eine lösbare Eingriffsstruktur oder eine lösbare Befestigungsstruktur ist.

4. Wirbelimplantat nach Anspruch 1, **dadurch gekennzeichnet, dass** ein Material der dreidimensionalen porösen Vorrichtung (120) ein biokompatibles Material ist.

5. Wirbelimplantat nach Anspruch 4, **dadurch gekennzeichnet, dass** das biokompatible Material Polyethylen, Polyurethan, Polyvinylalkohol, Nylon oder Silikon ist.

6. Wirbelimplantat nach Anspruch 1, **dadurch gekennzeichnet, dass** ein Material der dreidimensionalen porösen Vorrichtung (120) ein biologisch abbaubares Material ist.

7. Wirbelimplantat nach Anspruch 6, **dadurch gekennzeichnet, dass** das biologisch abbaubare Material Polymilchsäure, Gelatine, Alginate, Polyglykolsäure, ein Polyhydroxyfettsäureester oder Polyguilid ist.

8. Wirbelimplantat nach Anspruch 1, **dadurch gekennzeichnet, dass** die Porengröße der dreidimensionalen porösen Vorrichtung (120) 10 µm bis 3000 µm beträgt.

9. Wirbelimplantat nach Anspruch 1, **dadurch gekennzeichnet, dass** das medizinische Füllmaterial (700) ein medizinisches Suspension-Füllmaterial ist.

10. Wirbelimplantat nach Anspruch 1, **dadurch gekennzeichnet, dass** das Wirbelimplantat ferner einen Injektor (500) umfasst, der ein distales Ende aufweist, das mit dem proximalen Ende des Hohlrohrs (200) verbunden ist, und das medizinische Füllmaterial (700) durch den Injektor (500) in die dreidimensionale poröse Vorrichtung (120) eingefüllt wird.

11. Wirbelimplantat nach Anspruch 1, **dadurch gekennzeichnet, dass** das Wirbelimplantat ferner einen Injektor (500) und ein Verlängerungsrohr (600) umfasst, wobei ein distales Ende des Verlängerungsrohrs (600) und das proximale Ende des Hohlrohrs (200) lösbar miteinander verbunden sind, wobei ein proximales Ende des Verlängerungsrohrs (600) mit einem distalen Ende des Injektors (500) verbunden ist, und das medizinische Füllmaterial (700) durch den Injektor (500) in die dreidimensionale poröse Vorrichtung (120) eingespritzt wird.

12. Wirbelimplantat nach Anspruch 1, **dadurch gekennzeichnet, dass** das Wirbelimplantat ferner ein Hohlführungsrohr (300) umfasst, wobei ein proximales Ende des Hohlführungsrohrs (300) eine Einführöffnung (310) aufweist, wobei die Füllung (100) von der Einführöffnung (310) in das Hohlführungsrohr (300) eintritt und in die Wirbel durch Führung des Hohlführungsrohrs (300) eingeführt wird.

13. Wirbelimplantat nach Anspruch 12, **dadurch gekennzeichnet, dass** das Wirbelimplantat ferner ein Kompressionsteil (400) umfasst, wobei das Kompressionsteil (400) die dreidimensionale poröse Vorrichtung (120) abdeckt und komprimiert, bevor die dreidimensionale poröse Vorrichtung (120) in die Wirbel eingeführt wird, und sich von der dreidimensionalen porösen Vorrichtung (120) löst, nachdem die dreidimensionale poröse Vorrichtung (120) in das Hohlrohr (200) eingetreten ist.

## Revendications

1. Un implant vertébral comprenant:
un rembourrage (100) comprenant:
un dispositif de perfusion (110) ayant une extrémité proximale étant un port d'injection (112), le dispositif de perfusion (110) ayant au moins un port de perfusion (111) ; et
un dispositif poreux tridimensionnel (120) couvrant au moins une partie du dispositif de perfusion (110) et couvrant ledit au moins un port de perfusion (111);
un tube creux (200) ayant une extrémité distale reliée à l'extrémité proximale du rembourrage (100) par une structure détachable (140) ; et
un matériau de remplissage médical (700) étant introduit dans le dispositif poreux tridimensionnel (120) via le tube creux (200) et le dispositif de perfusion (110);
**caractérisé en ce que** le dispositif poreux tridimensionnel (120) est une structure tridimensionnelle à pores connectés, et **en ce que** le dispositif poreux tridimensionnel (120) est capable de se dilater automatiquement après avoir été comprimé.

2. L'implant vertébral selon la revendication 1, **caractérisé en ce que** le rembourrage (100) comprend en outre au moins une pièce de fixation (130) pour fixer le dispositif poreux tridimensionnel (120) au dispositif de perfusion (110).

3. L'implant vertébral selon la revendication 1, **caractérisé en ce que** la structure détachable (140) est une structure de vis détachable, une structure d'engagement détachable ou une structure de fixation détachable.

4. L'implant vertébral selon la revendication 1, **caractérisé en ce que** le matériau du dispositif poreux tridimensionnel (120) est un matériau biocompatible.

5. L'implant vertébral selon la revendication 4, **caractérisé en ce que** le matériau biocompatible est du polyéthylène, du polyuréthane, de l'alcool polyvinylique, du nylon ou du silicone.

6. L'implant vertébral selon la revendication 1, **caractérisé en ce que** le matériau du dispositif poreux tridimensionnel (120) est un matériau biodégradable.

7. L'implant vertébral selon la revendication 6, **caractérisé en ce que** le matériau biodégradable est de l'acide polylactique, de la gélatine, de l'alginate, de l'acide polyglycolique, un ester d'acide gras polyhydroxylé ou du polyguilide.

8. L'implant vertébral selon la revendication 1, **caractérisé en ce que** la taille des pores du dispositif poreux tridimensionnel (120) est de 10µm à 3000µm.

9. L'implant vertébral selon la revendication 1, **caractérisé en ce que** le matériau de remplissage médical (700) est un matériau de remplissage médical en suspension.

10. L'implant vertébral selon la revendication 1, **caractérisé en ce que** l'implant vertébral comprend en outre un injecteur (500) ayant une extrémité distale reliée à l'extrémité proximale du tube creux (200), et le matériau de remplissage médical (700) est introduit dans le dispositif poreux tridimensionnel (120) par l'injecteur (500).

11. L'implant vertébral selon la revendication 1, **caractérisé en ce que** l'implant vertébral comprend en outre un injecteur (500) et un tube d'extension (600), une extrémité distale du tube d'extension (600) et l'extrémité proximale du tube creux (200) sont reliées de manière détachable, une extrémité proximale du tube d'extension (600) est reliée à une extrémité distale de l'injecteur (500), le matériau de remplissage médical (700) est injecté dans le dispositif poreux tridimensionnel (120) par l'injecteur (500).

12. L'implant vertébral selon la revendication 1, **caractérisé en ce que** l'implant vertébral comprend en outre un tube de guidage creux (300), une extrémité proximale du tube de guidage creux (300) ayant un port d'introduction (310), le rembourrage (100) entre dans le tube de guidage creux (300) à partir du port d'introduction (310) et est inséré dans les vertèbres par guidage du tube de guidage creux (300).

13. L'implant vertébral selon la revendication 12, **caractérisé en ce que** l'implant vertébral comprend en outre une pièce de compression (400), la pièce de compression (400) couvre et comprime le dispositif poreux tridimensionnel (120) avant que le dispositif poreux tridimensionnel (120) ne soit inséré dans les vertèbres, et se détache du dispositif poreux tridimensionnel (120) après que le dispositif poreux tridimensionnel (120) soit entré dans le tube creux (200).
